# EUROPEAN PATENT SPECIFICATION

(11) **EP 4 349 306 B1**
(45) Date of publication and mention of the grant of the patent: **28.10.2026**
(21) Application number: 24155012.8
(22) Date of filing: 18.01.2022
(51) Int. Cl.: A61F 5/44, A61F 5/442, A61F 5/455

(54) **VARIABLE FIT FLUID COLLECTION DEVICES**
FLÜSSIGKEITSSAMMELVORRICHTUNGEN MIT VARIABLER PASSFORM, SYSTEME UND VERFAHREN
DISPOSITIFS, SYSTÈMES ET PROCÉDÉS DE COLLECTE DE FLUIDE À AJUSTEMENT VARIABLE

(30) Priority: 19.01.2021 US 202163138878 P
(43) Date of publication of application: 10.04.2024
(62) Document number(s) of the earlier application(s) in accordance with Art. 76 EPC: 22703201.8 / 4 110 247
(73) Proprietor: Purewick Corporation, El Cajon, California 92020 (US)
(72) Inventor: DAVIS, Kathleen, Atlanta, GA 30307 (US); GRUPP, Katie, Alpharetta, GA 30009 (US); STARKIE, Joanna, Cambridge, CB22 7GG (GB); LIM SARRIAS, Merissa, Hauxton, CB22 5HZ (GB); TRIGGS, Emily Lucy, Cambridge, CB22 3LG (GB); WHITTOME, Samuel Edmund, Cambridge, CB1 9GQ (GB)
(74) Representative: Hoffmann Eitle

(56) References cited:
- EP-A1- 0 068 712
- US-A- 5 282 795
- US-A1- 2018 228 642

## Description

### BACKGROUND

An individual may have limited or impaired mobility such that utilizing a restroom is challenging or impossible. For example, the individual may have a condition, had a surgery, or a have disability that impairs mobility. Additionally, bodily fluid collection from the individual may be needed for monitoring purposes or clinical testing.

Bedpans and other common urine collection devices suffer from various drawbacks, such as difficulty of use, embarrassing visibility of urine in the device, and some devices cannot be worn at all times, thereby making spills and leakage a risk. Thus, users and manufacturers of patient care beds continue to seek new and improved devices, systems, and methods to prevent patient's clothing and beds from becoming soiled. US 2018/228642 A1 discloses a device for collecting urine having at least one layer for drawing urine discharged from the body into an interior cavity, an external covering that covers a portion of the at least one layer, and at least one fenestration for receiving urine, wherein the fenestration is a portion of the fluid collection assembly that is uncovered by the external covering. The device comprises a cap enclosing a first end of the assembly, a tube having a first end in fluid communication with the cap, and a shape retaining element configured to conform the assembly to a curved configuration and maintain the curved configuration until the configuration is adjusted.

### SUMMARY

Embodiments disclosed herein are related a fluid collection device for collecting fluid from wearers, where the fluid collection devices include a retention means for maintaining the position of the fluid collection device with respect to the wearer.

The present invention relates to a fluid collection device according to claim 1.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1** is a front view of a fluid collection device for females, according to an embodiment.
**FIG. 2** is a cross-sectional view of the fluid collection device of **FIG. 1****.**
**FIGS. 3** and **4** are cross-sectional views of a deformable conduit, according to an embodiment.
**FIG. 5** is an isometric view of a portion of a deformable conduit, according to an embodiment.
**FIG. 6** is a close-up partial cut-away view of the deformable conduit of **FIG. 5****.**
**FIG. 7** is a side view of a fluid collection device changing from a compressed to an uncompressed state, according to an embodiment.
**FIGS. 8** and **9** are isometric views of a fluid collection device, according to an embodiment.
**FIGS. 10A and 10B** **are** illustrations of the fluid collection device of **FIGS. 8** and **9** in cross-section during positioning.
**FIGS. 11** and **12** are top views of a fluid collection device having a mucoadhesive applied thereto, according to an embodiment.
**FIG. 13** is a block diagram of a system for collecting fluid, according to an embodiment.
**FIG. 14** is a flow diagram of a method for collecting fluid, according to an embodiment.

### DETAILED DESCRIPTION

Embodiments disclosed herein are related to devices for collecting fluid with fluid collection devices having means for retaining position with respect to a wearer. Systems, and methods for collecting fluid are also disclosed. The devices, systems, and methods disclosed herein include fluid collection devices having a fluid impermeable barrier defining an interior chamber therein, a porous material disposed at least partially within the interior chamber, and a conduit extending into the interior chamber to remove fluids collected within the interior chamber by the porous material. The retention means allow the porous material of the fluid collection device to remain positioned on or over the urethra of the wearer during use. The various retention means provide one or more of conformational rigidity, conformability to wearer anatomy, friction fit with wearer anatomy, or mucoadhesion with wearer anatomy.

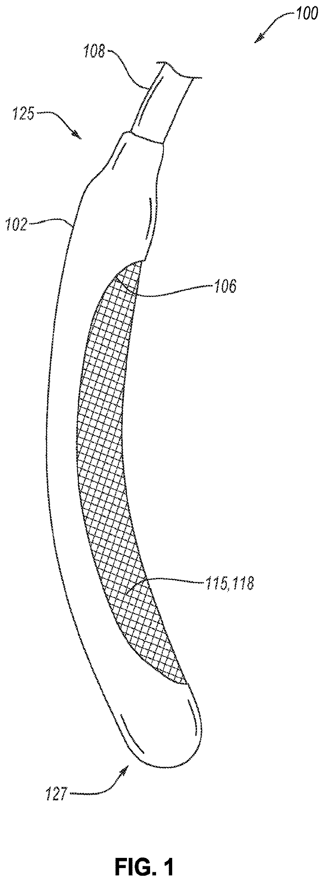

In a first example, a fluid collection device includes a selectively deformable spine in or around the conduit to maintain a shape of the fluid collection device. **FIG. 1** is a front view of a fluid collection device 100 for females, according to an embodiment. The fluid collection device 100 includes a fluid impermeable barrier 102 defining an interior chamber therein and an opening 106. The fluid collection device 100 includes porous material 115 disposed in the interior chamber and exposed through the opening 106. The fluid collection device 100 includes a conduit 108 (*e.g.,* drainage tube) disposed in the interior chamber and entering from the first end region 125 and extending to the second end region 127. The fluid collection device 100 is sized, shaped, and constructed to collect urine or other fluids from the urethra of the wearer.

The fluid impermeable barrier 102 may be formed of any suitable fluid impermeable material(s), such as a fluid impermeable polymer (*e.g*., silicone, polypropylene, polyethylene, polyethylene terephthalate, thermoplastic elastomer(s), a polycarbonate, etc.), a metal film, natural rubber, another suitable material, or combinations thereof. As such, the fluid impermeable barrier 102 substantially prevents the fluid(s) from passing therethrough. The fluid impermeable barrier 102 at least partially defines the interior chamber (e.g., interior region) therein and the opening 106. For example, the inner surface(s) of the fluid impermeable barrier 102 at least partially defines the interior chamber within the fluid collection device 100. The fluid impermeable barrier 102 at least temporarily retains the fluid(s) in the interior chamber. One or more portions of at least the outer surface of the fluid impermeable barrier 102 may be formed from a soft and/or smooth material, thereby reducing chaffing.

The fluid impermeable barrier 102 may be tubular (ignoring the opening), such as substantially cylindrical (as shown), oblong, prismatic, flattened tube, or any other extruded shape *(e.g.,* a tube having multiple flanges). The fluid impermeable barrier 102 may be sized to fit between the legs of a wearer. During use, the outer surface of the fluid impermeable barrier 102 may at least partially contact the wearer.

The opening 106 provides an ingress route for fluids to enter the interior chamber. The opening 106 may be defined by the fluid impermeable barrier 102, such as by an inner edge of the fluid impermeable barrier 102. For example, the opening 106 is formed in and extends through the fluid impermeable barrier 102, from the outer surface to the inner surface, thereby enabling fluid(s) to enter the interior chamber from outside of the fluid collection device 100. The opening 106 is located and shaped to be positioned adjacent to a wearer's urethra while the device is in use. At least a portion of porous material(s) 115 disposed in the interior may be exposed through the opening 106 to allow fluids to move inwardly into the interior chamber, such as via one or more of permeation, suction, or wicking.

The fluid collection device 100 may be positioned proximate to the urethra and urine may enter the interior chamber via the opening 106. When in use, the opening 106 may be elongated, extending from a first location below the urethra to a second location above the urethra (*e.g.,* at or near the top of the vaginal opening or the pubic region). The opening 106 may exhibit an elongated shape because the space between the legs of a wearer is relatively narrow when the legs of the wearer are closed, thereby only permitting the flow of the fluid(s) along a path that corresponds to the elongated shape of the opening 106 (*e.g.,* longitudinally extending opening). The opening 106 in the fluid impermeable barrier 102 may exhibit a length that is measured along the longitudinal axis of the fluid collection device 100 that may be at least about 10% of the length of the fluid collection device 100, such as about 10% to about 95% of the length of the fluid collection device 100. The opening 106 in the fluid impermeable barrier 102 may exhibit a width that is measured transverse to the longitudinal axis of the fluid collection device 100 and may be at least about 10% of the circumference of the fluid collection device 100, such as about 10% to about 75% of the circumference of the fluid collection device. The opening 406 may be longitudinally oriented (*e.g*., having a major axis parallel to the longitudinal axis of the device 100.

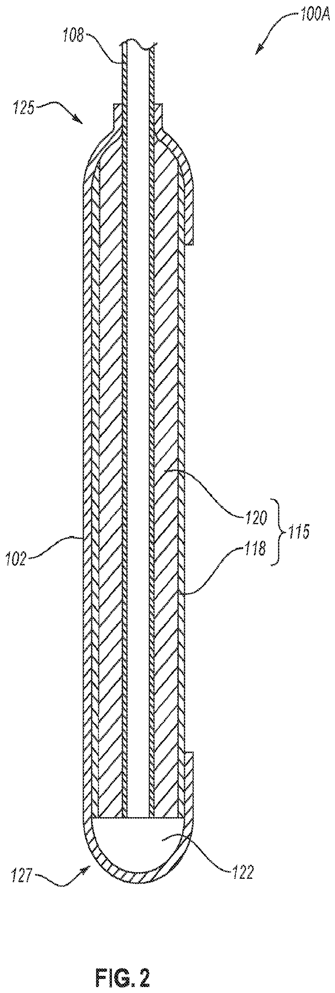

The fluid collection device 100 includes the porous material 115 disposed in the interior chamber. **FIG. 2** is a cross-sectional view of the fluid collection device 100. The fluid collection device 100 includes the porous material 115 including a fluid permeable support 120 within a fluid permeable membrane 118 and a conduit 108 extending through the porous material 115. The porous material 115 includes a material that allows fluids to flow therethrough. For example, the porous material 115 may be configured to wick any fluid away from the opening 106, thereby preventing the fluid from escaping the interior chamber. The permeable properties of the porous material referred to herein may be wicking, capillary action, diffusion, or other similar properties or processes, and are referred to herein as "permeable" and/or "wicking." Such "wicking" may not include absorption of fluid into the porous material. Put another way, substantially no absorption of fluid into the porous material may take place after the material is exposed to the fluid and removed from the fluid for a time. While no absorption is desired, the term "substantially no absorption" may allow for nominal amounts of absorption of fluid into the porous material (e.g., absorbency), such as less than about 10 wt% of the dry weight of the porous material, less than about 5 wt%, less than about 3 wt%, less than about 1 wt%, or less than about 0.5 wt% of the dry weight of the porous material 115. The porous material 115 may also wick the fluid generally towards a central region or reservoir of the interior chamber.

The porous material 115 may include more than one material arranged concentrically within the interior chamber, such as gauze disposed over spun plastic fibers (*e.g.,* spun nylon fibers). For example, the porous material 115 may include one or more of a fluid permeable membrane 118 or a fluid permeable support 120 disposed thereunder. The fluid permeable membrane 118 may include any porous material or a material that may wick the fluid. For example, the fluid permeable membrane 118 may include fabric, such as a gauze (*e.g.,* a silk, linen, or cotton gauze), another soft fabric, or another smooth fabric. The fluid permeable membrane 118 may include spun plastic fibers (*e.g.,* spun nylon fibers), such as a spun plastic mat or bed. Forming the fluid permeable membrane 118 from gauze, soft fabric, and/or smooth fabric may reduce chafing caused by the fluid collection device 100 against a wearer.

The fluid collection device 100 may include the fluid permeable membrane 118 disposed in the interior chamber. For example, the fluid permeable membrane 118 may cover at least a portion (*e.g.,* all) of the opening 106.

The porous material 115 of the fluid collection device 100 may include the fluid permeable support 120 disposed in the interior chamber below the fluid permeable membrane 118. The fluid permeable support 120 may be composed to support the fluid permeable membrane 118 since the fluid permeable membrane 118 may be formed from a foldable, flimsy, or otherwise easily deformable material. For example, the fluid permeable support 120 may be positioned such that the fluid permeable membrane 118 is disposed between the fluid permeable support 120 and the fluid impermeable barrier 102. As such, the fluid permeable support 120 may support and maintain the position of the fluid permeable membrane 118 thereon. The fluid permeable support 120 may include any porous, fluid permeable material, such as a material that may wick the fluid. For example, the fluid permeable support 120 may include any of the fluid permeable membrane materials disclosed herein. The fluid permeable support 120 may be formed from any fluid porous material that is less deformable than the fluid permeable membrane 118, such as any of the materials disclosed herein for the fluid permeable membrane 118, in a more dense or rigid form. In some examples, the fluid permeable support 120 may include a porous polymer (e.g., nylon, polyester, polyurethane, polyethylene, polypropylene, etc.) structure, an open cell foam, or spun plastic fibers (*e.g.,* nylon fibers). In some examples, the fluid permeable membrane 118 may include gauze and the fluid permeable support 120 may include spun nylon fibers. In some examples, the fluid permeable support 120 may be formed from fabric, felt, gauze, or combinations thereof. In some examples, the fluid permeable support 120 may be formed from a natural material, such as cotton, wool, silk, or combinations thereof. In such examples, the material may have a coating to prevent or limit absorption of fluid into the material, such as a water repellent coating. In some examples, the fluid permeable support 120 may be omitted from the fluid collection device 100. In some examples, the fluid permeable membrane 118 may be optional. For example, the porous material 115 may include only the fluid permeable support 120.

The fluid permeable support may have a greater permeability or a greater ability to wick fluids than the fluid permeable membrane 118, such as to move the fluid inwardly from the outer surface of the fluid collection device 100 through the permeable membrane 118 and into the fluid permeable support. In some examples, the permeability or the wicking ability of the fluid permeable support and the fluid permeable membrane 118 may be substantially the same.

The fluid impermeable barrier 102, the porous material 115 may be sized and shaped to have the conduit 108 at least partially disposed in the interior chamber. For example, at least one of the fluid permeable membrane 118 and the fluid permeable support 120 may be configured to form a space that accommodates the conduit 108. The fluid impermeable barrier 102 may define an aperture sized to receive the conduit 108 therethrough. The aperture may be sized and shaped to form an at least substantially fluid tight seal against the conduit 108 thereby substantially preventing the fluid(s) from escaping the interior chamber. The fluid collected in the fluid collection device 100 may be removed from the interior chamber via the conduit 108.

The porous material 115 may extend across at least a portion *(e.g.,* all) of the opening 106. At least a portion of the porous material 115 may be exposed to an environment outside of the interior chamber through the opening 106. The fluid permeable membrane 118 and the fluid permeable support 120 may at least substantially completely fill the portions of the interior chamber that are not occupied by the conduit 108. In another example, the fluid permeable membrane 118 and the fluid permeable support 120 may not substantially completely fill the portions of the interior chamber that are not occupied by the conduit 108. In such an example, the fluid collection device 100 includes reservoir 122 in the interior chamber. The reservoir 122 is defined between the fluid impermeable barrier 102 and the porous material 115 in the interior chamber. The reservoir 122 may be located in a portion of the fluid collection device 100 expected to be positioned in a gravimetrically low point of thereof when worn by a user. While depicted in the second end region 127, the reservoir 122 may be located in any portion of the interior chamber such as the first end region 125.

As shown in **FIG. 1****,** the fluid collection device 100 includes conduit 108 (*e.g.,* drainage tube) which extends in the interior chamber. The conduit 108 may include a flexible material such as polymer tubing (*e.g*., medical tubing) material. Such polymer tubing material may include a thermoplastic elastomer, polyvinyl chloride, ethylene vinyl acetate, polytetrafluoroethylene, etc., tubing. In some examples, the conduit 108 may include silicon or latex. In some examples, the conduit 108 may include one or more portions that are resilient, such as to by having one or more of a diameter or wall thickness that allows the conduit 108 to be flexible. In some examples, the conduit 108 may be frosted or opaque (*e.g*., black) to obscure visibility of the fluid(s) therein.

The conduit 108 includes an inlet at a first end region and an outlet at a second end region positioned downstream from the inlet. The conduit 108 may extend into the interior chamber to any point therein. For example, the conduit 108 may be inserted into the interior chamber at the first end region 125 of the fluid collection device 100 and extend therethrough to the second end region 127. The conduit 108 may extend into the fluid impermeable barrier 102 from the first end region 125 through to the second end region 127 to a point proximate to the reservoir 122 such that the inlet is in fluid communication with the reservoir 122. In some examples (not shown), the conduit 108 may enter the interior chamber in the second end region 127 and the inlet of the conduit 108 may be disposed in the second end region 127 (*e.g.,* in the reservoir 122 or flush with fluid impermeable barrier 102). The fluid collected in the reservoir 122 may be removed from the interior chamber via the conduit 108. In some examples, the inlet may be disposed at the end of the porous material 115 in the second end region 127, such as flush with the end of the fluid permeable support 120. In some examples, the inlet of the conduit 108 may be disposed within the fluid permeable support 120 such between first end region 125 and the second end region 127.

Other examples of fluid impermeable barriers, porous materials, chambers, conduits and their shapes and configurations are disclosed in U.S. Patent Application No. 15/612,325 filed on June 2, 2017; U.S. Patent Application No. 15/260,103 filed on September 8, 2016; and U.S. Patent No. 10,226,376 filed on June 1, 2017.

The conduit 108 provides a selectively deformable spine within the fluid collection device 100. Upon manual manipulation, the conduit 108 may hold the fluid collection device 100 in a specific shape, such as the illustrated curved shape. For example, the fluid collection device 100 is able to deform to the curvature of the body between the glutes and the labia by manipulating the conduit 108. The reduction in tension along the spine of the fluid collection device 100 provided by the conduit 108 reduces the likelihood of detachment or misalignment of the fluid collection device 100 from the urethra (*e.g.,* on top of the labia).

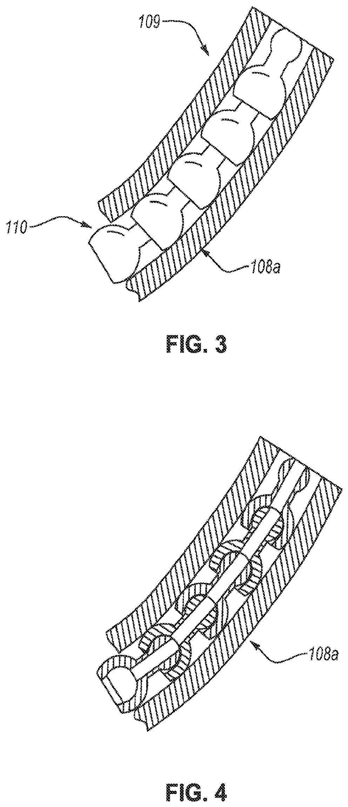

Disclosed below are examples for the conduit 108 to provide a selectively deformable spine in the fluid collection device 100. **FIGS. 3** and **4** are cross-sectional views of a deformable conduit 108a, according to an embodiment. As shown in **FIG. 3****,** the deformable conduit 108a includes tubing 109 disposed over flexible gooseneck tubing 110. The gooseneck tubing 110 may be a modular hose that is capable of manipulation responsive to external forces and rigid retention of a selected configuration after manipulation. For example, the gooseneck tubing 110 may include a ball and socket construction such as a Loc-Line^{®} type modular hose or the like. The size of the balls and sockets of the gooseneck tubing 110 provide a selected fit therebetween (*e.g*., slip fit, interference fit, etc.) to provide a selected amount of resistance to deformation. Accordingly, the deformable conduit 108a may be selectively shaped (*e.g*., deformed) and retain the selected shape or conformation. In some examples, the gooseneck tubing 110 may be constructed of polymer balls and sockets.

As shown in **FIG. 4****,** the gooseneck tubing 110 includes an interior passageway therein for enabling fluid to move therethrough. The interior passageway is formed by the interior walls of the balls and sockets of the gooseneck tubing, which have a selected bore size therein. The fluids (*e.g*., urine) collected in the fluid collection device having the deformable conduit 108a flow though the interior passageway of the deformable conduit 108a as the fluid drains therefrom.

The gooseneck tubing 110 is not fluid tight. Accordingly, the tubing 109 therearound may help retain fluids within the deformable conduit 108a. The tubing 109 may be relatively soft and flexible compared to conventional drainage tubing. The tubing 109 provides a cushion between the wearer and the gooseneck tubing 110. The tubing 109 may be constructed of a polymer such as a thermoplastic elastomer, polyvinyl chloride, ethylene vinyl acetate, polytetrafluoroethylene, etc., or combinations of any of the foregoing. In some examples, the tubing 109 may include silicone or latex. The tubing 109 may have a wall thickness of at least about 1 mm, such as about 1 mm to about 5 mm, or less than 10 mm. The deformable conduit 108a may include tubing 109 having a thickness that prevents wearers from feeling the gooseneck tubing 110 therebelow.

The diameter or greatest dimension of the gooseneck tubing 110 may be about 13 mm or less, such as about 5 mm to about 13 mm, about 5 mm to about 8 mm, about 8 mm to about 13 mm, or more than about 8 mm. In some examples, the diameter or greatest dimension of the deformable conduit 108a (including the outer dimensions of the tubing 109 disposed over the gooseneck tubing 110) may be about 13 mm or less, such as about 5 mm to about 13 mm, about 5 mm to about 8 mm, about 8 mm to about 13 mm, or more than about 8 mm. Fluid collection devices incorporating the deformable conduit 108a may include porous material having a thickness that prevents wearers from feeling the gooseneck tubing 110 of the deformable conduit 108a therebelow.

The gooseneck tubing 110 extends only along a portion of the deformable conduit 108a inside of the interior chamber. The deformable conduit 108a may be used in any of the examples of fluid collection devices disclosed herein.

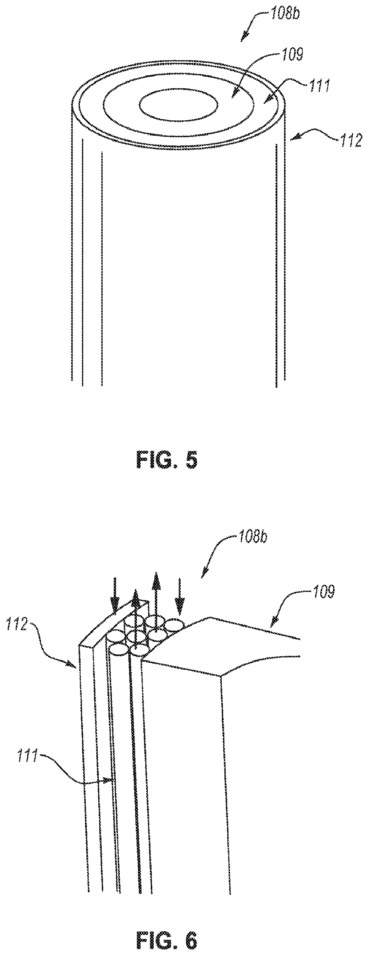

Different deformable conduits may be utilized in fluid collection devices. **FIG. 5** is an isometric view of a portion of the deformable conduit 108b, according to an embodiment. **FIG. 6** is a close-up partial cut-away view of the deformable conduit 108b of **FIG. 5****.** The deformable conduit 108b may include a plurality of fibers 111 sandwiched between tubing 109 and an outer tube 112. The plurality of fibers 111 may include polymer fibers or wires. The plurality of fibers may include wires including one or more of polycarbonate, polyethylene, polyvinylchloride, polypropylene, polyolefin, fluorinated polymers (polytetrafluoroethylene), polyamide, or the like. The width of the fibers or wires may be 0.1 mm or more, such as 0.1 mm to 3 mm, 0.1 mm to 1 mm, 1 mm to 3 mm, less than 3 mm, or less than 1 mm. As the deformable conduit 108b is bent, the polymer fibers move past each other and the friction therebetween holds them in the new position to prevent spring back. The plurality of fibers may be packed between the outer tube 112 and the tubing 109 with a selected spacing to provide a selected fit therebetween, such as a slip fit, a tension fit, an interference fit, or the like. The tightness of the packing within the plurality of fibers 111 can provide fine tuning of the stiffness of the deformable conduit 108b. Accordingly, the deformable conduit 108b may retain a selected shape according to manual manipulation and may resist forces of a selected magnitude to change the shape.

The tubing 109 may be as disclosed herein with respect to the deformable conduit 108a. The outer tube 112 may be similar or identical to the conduit 108 or the tubing 109, in one or more aspects. For example, the outer tubing 112 may including a polymer conduit, such as thermoplastic elastomer, polyvinyl chloride, ethylene vinyl acetate, polytetrafluoroethylene, silicone, latex, or combinations thereof.

The plurality of fibers 111, tubing 109, and outer tube may extend only along a portion of the deformable conduit 108b, such as the portion inside of the interior chamber or from inside of the interior chamber to a distance outside of the first end region of the fluid collection device containing the same, such as at least 1 cm past the aperture through which the conduit 108b is inserted into the fluid collection device. The deformable conduit 108b may be used in any of the examples of fluid collection devices disclosed herein.

The deformable conduits 108a and 108b may be sized and shaped to conform the fluid collection devices carrying the same to the anatomy of a wearer along the sagittal plane of the wearer, such as in the pelvic region. Such examples provide fluid collection devices that can be manipulated to a selected shape and may resist changing from the selected shape, such as from movement of the wearer.

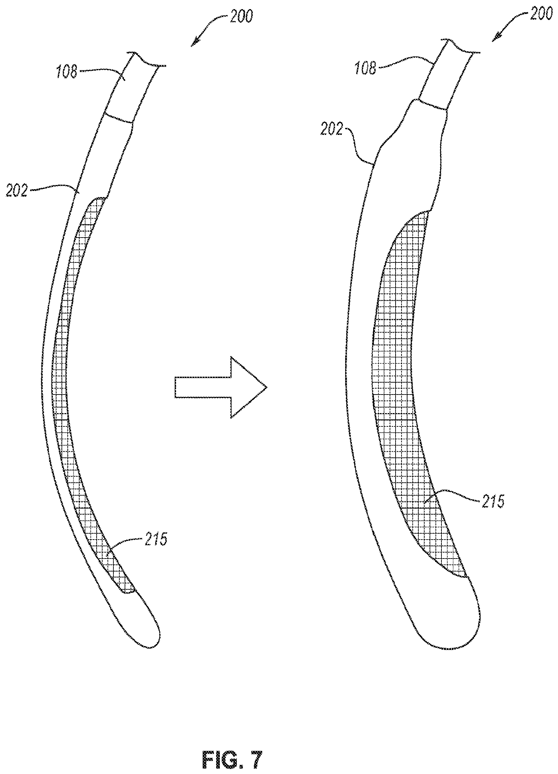

In some examples, the fluid collection device may be at least partially held in place by a resiliently compressible design. **FIG. 7** is a side view of fluid collection device 200 changing from a compressed to an uncompressed state. The fluid collection device 200 includes the fluid impermeable barrier 202, the porous material 215, and the conduit 108 extending therethrough. The fluid collection device 200 is resiliently compressible and may at least partially hold a shape responsive to pressure applied thereto.

The porous material 215 includes a soft, resilient foam, such as a viscoelastic foam (e.g., memory foam or low-resilience polyurethane). Accordingly, the porous material 215 is compressible. The resilient foam of the porous material 215 is an open cell foam to allow fluids to pass therethrough. The porous material 215 may include one or more of the fluid permeable membrane or the fluid permeable support. The resilient foam of the porous material 215 may be used alone as a fluid permeable membrane or the fluid permeable support. In the latter case, a fluid permeable membrane, such as any of those disclosed herein (*e.g.,* gauze) may be disposed over the resilient foam.

The fluid impermeable barrier 202 is a soft malleable material capable of being deformed and springing back to an original shape, either alone or as forced by the resilient foam of the porous material 215. The fluid impermeable barrier 202 may be similar or identical to the fluid impermeable barrier 102 in one or more aspects. The fluid impermeable barrier 202 may include silicone, rubber, or a polymer as disclosed herein. The fluid impermeable barrier 202 may be thin layer directly attached to the porous material 215, such as a coating thereon. For example, the fluid impermeable barrier may be a waterproof polyurethane laminate fabric or the like, which may be welded directly to the foam or wrapped therearound.

The conduit 108 may be made of a relatively malleable (compared to conventional medical drainage tubing), fluid-tight material. For example, the conduit 108 may be made of one or more of polyethylene, polyvinyl chloride, latex, silicone, or a mixture thereof. Higher amounts of silicone provide a softer, more malleable conduit 108. The conduit 108 is malleable responsive to pressure such that the fluid collection device 200 is not shaped by the stiffness of the conduit 108.

In use, the fluid collection device 200 is compressed and inserted or pressed against the anatomy of the wearer. As the fluid collection device 200 returns to an expanded shape, the fluid collection device 200 at least partially conforms to the anatomy of the wearer. Accordingly, the fluid collection device 200 provides an improved anatomical fit to increase comfort, maintain position on the wearer, and decrease leakage over less conformable materials and devices. The resiliently compressible components (*e.g.,* the resilient foam of the porous material 215) provides a means of retaining the fluid collection device 200 in position on the wearer. The deformable conduits 108a and 108b may be used with the fluid collection device 200.

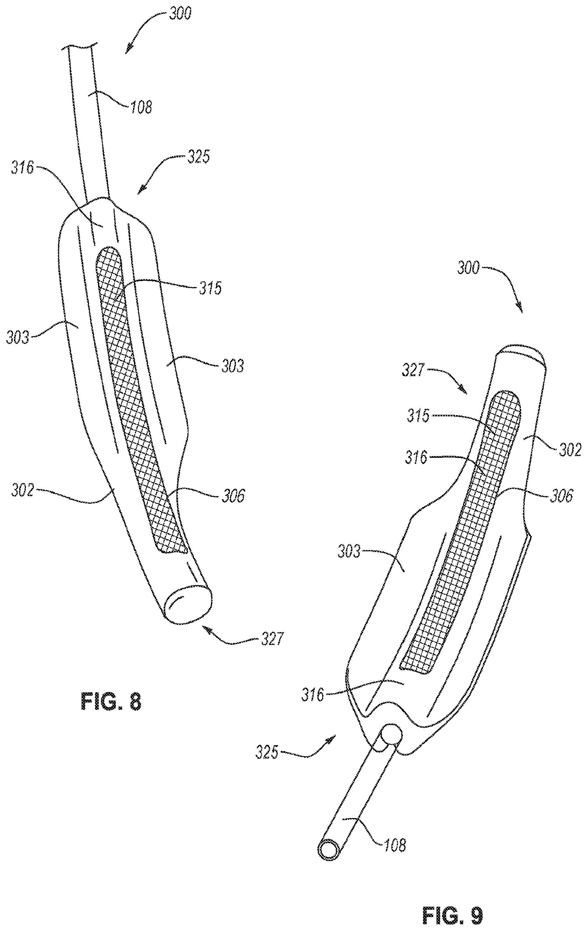

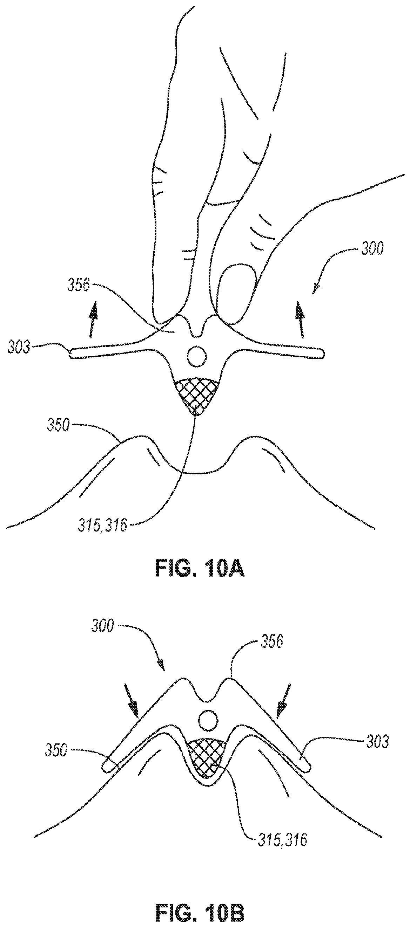

In some examples, the shape of the fluid collection device is generally preformed to the shape of labia and can be manipulated from a closed state to an open state by compression of a portion thereof. **FIGS. 8** and **9** are isometric views of fluid collection device 300, according to an embodiment. **FIGS. 10A and 10B** **are** illustrations of the fluid collection device 300 in cross-section during positioning. The fluid collection device 300 includes fluid impermeable barrier 302 having labial flanges 303 extending outwardly from a longitudinal axis thereof, porous material 315, the conduit 108, and lobes 356 extending longitudinally along the back of the fluid collection device 300.

The fluid impermeable barrier 302 includes an outer surface and an inner surface that defines an interior chamber therein. The fluid impermeable barrier 302 includes an opening 306. The fluid impermeable barrier 302 at least partially defines the labial flanges 303 and the lobes 356. The porous material 315 is at least partially disposed in the interior chamber and protrudes therefrom through the opening 306. The conduit 108 extends into the interior chamber via fluid impermeable barrier, such as into the porous material (e.g., to a reservoir in the interior chamber). The porous material 315 may be similar or identical to any of the porous material(s) disclosed herein, such as having a fluid permeable membrane and a fluid permeable support. The fluid impermeable barrier 302 is formed of a fluid impermeable material such as any of the fluid impermeable materials disclosed herein.

The labial flanges 303 are shaped with longitudinally extending valleys sized to at least partially accommodate labia majora therein. The labial flanges 303 may be at least partially rigid. The fluid impermeable barrier 302 converges to an apex 316 along the longitudinal axis in the first end region 325. The porous material 315 also includes an apex 316 along the longitudinal axis. Accordingly, the apex 316 may be positioned between the labia majora and the valleys accommodate the labia majora during use. The outer surfaces of the labial flanges 303 (*e.g.,* on the wearer facing surface) may be textured or tacky for frictional engagement with the labia of the wearer. The longitudinal shape of the fluid collection device or portions thereof may be at least slightly arcuate to complement the shape of wearer anatomy.

On the back of the fluid collection device 300, the fluid impermeable barrier 302 defines the lobes 356. The lobes 356 have a valley therebetween. The lobes may longitudinally extend along at least a portion of the back side of the fluid collection device 300. The lobes 356 may be pinched inwardly, as shown in **FIGS. 10A and 10B****,** to open the labial flanges 303 and expose the apex 316. For example, the lateral ends of the labial flanges 303 rotate away from the apex 316 when the lobes 356 are pinched due to tension applied across the fluid impermeable barrier 302. The apex 316 may be inserted between the labia majora 350. Upon release, the lateral ends of the labial flanges 303 rotate back toward the apex 316 to engage the outer surface of the labia majora 350 with the valleys formed between the labial flanges 303 and the apex 316 at least partially containing the labia majora therein. Accordingly, the apex 316 of the porous material 315 may be positioned closer to the urethra of the wearer than cylindrical devices. The labial flanges 303 may hold onto the labia majora, by inward pressure or pinching after the inward pressure on the lobes 356 is released. Accordingly, an additional means of retention is provided by the shape of the fluid collection device 300.

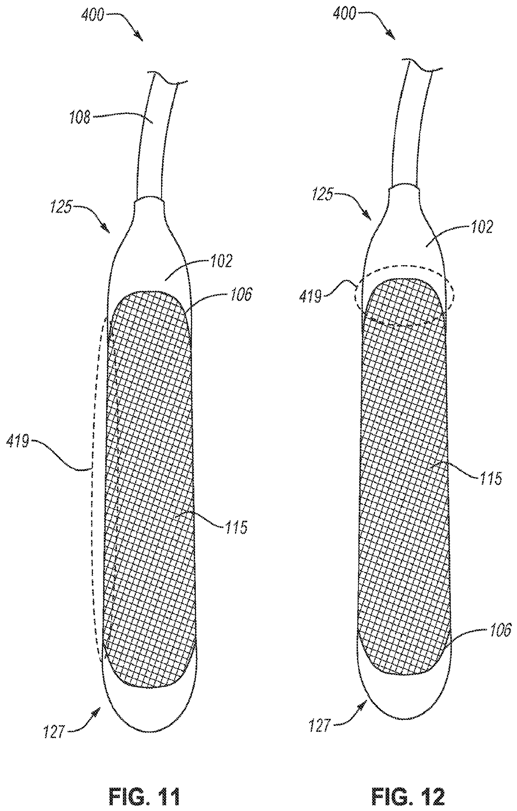

In some examples, a fluid collection device may use a mucoadhesive to increase engagement with the wearer. **FIGS. 11** and **12** are top views of a fluid collection device 400 having a mucoadhesive applied thereto, according to an embodiment. The fluid collection device 400 includes the fluid impermeable barrier 102 defining the outer surface and the inner surface which defines the interior chamber of the fluid collection device 400. The fluid collection device 400 includes the porous material 115. The porous material 115 is exposed through the opening 106 in the fluid impermeable barrier 102. The fluid collection device 400 includes the conduit 108 extending into the fluid impermeable barrier 102 and porous material 115 from the first end region to the second end region of the fluid collection device 400. The fluid collection device includes a mucoadhesive applied to at least a portion of the fluid impermeable barrier 102 in the regions 419.

The mucoadhesive in the region 419 adds a tackifier to the fluid impermeable barrier 102 which provides shear resistance with a nearly no impact on peel strength. The mucoadhesive may include a gum, a gel, a polymer, a tape, or combinations thereof. For example, the mucoadhesive gum may include a Tmarind/Xanthum gum or the like. The mucoadhesive in gel form may include a Gantrez/Sodium alginate gel or the like. In some examples, the mucoadhesive includes a tape having surface modifiers thereon, a low tack adhesive with a tackifying additive integrated to the fluid impermeable barrier. Other gums, gels, or polymers may be used. The mucoadhesive may be disposed on one or more portions of the fluid collection device 400, such as on the fluid impermeable barrier 102 in region 419 as shown in **FIG. 11** or on the porous material 115 near the top of the opening in region 419 as shown in **FIG. 12****.**

As shown in **FIG. 11****,** the region 419 may be along the sides of the opening through which the porous material 115 is exposed. In such examples, the mucoadhesive may provide increased engagement with the inner legs (thighs) or labia of the wearer.

As shown in **FIG. 12****,** the region 419 may be at the top of the opening through which the porous material 115 is exposed. In such examples, the mucoadhesive may be disposed on the fluid impermeable barrier 102 and/or porous material 115 at the top of the opening in region 419. In such examples, the mucoadhesive provides increased engagement with the pubic region of the wearer, thereby keeping the opening (and porous material 115 therein) over the urethra of the wearer.

The mucoadhesive included on the fluid collection device 400 provides and additional retention means to maintain the positioning of the fluid collection device 400 with respect to the wearer. By limiting the area that the mucoadhesive is disposed on, the wearer, the wearer's clothing, and bedding may be prevented from contamination by the mucoadhesive. Accordingly, the mucoadhesive retains the position of the fluid collection device on the wearer without introducing large amounts of adhesive onto the wearer.

During use, the fluid collection device(s) disclosed herein may be positioned such that the opening of the fluid collection device through which the porous material is exposed is disposed on or over the urethra of the wearer. The fluid collection device may receive bodily fluids (*e.g.,* urine) from the urethra into the internal chamber. At least some of the bodily fluids may be removed from the urethra via the porous material (*e.g*., via wicking, absorptions, adsorption, etc.). The bodily fluids may flow towards the open end of the conduit in the reservoir (e.g., in the gravimetrically low point of the device) through the porous material in the interior chamber. The bodily fluids may be removed from the fluid collection device via the conduit. Suction force may be introduced into the interior chamber via the conduit responsive to suction (e.g., vacuum) force applied to the conduit by a vacuum source. The bodily fluids removed from the fluid collection device may be stored in the fluid storage container operably coupled to the vacuum source.

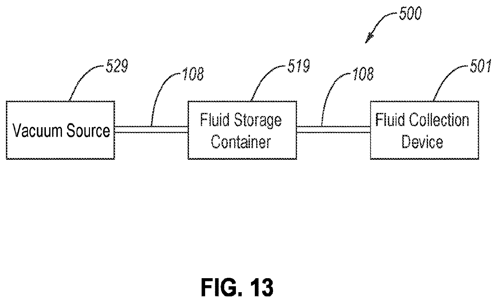

The fluid collection devices disclosed herein may be part of a fluid collection system. **FIG. 13** is a block diagram of a system 500 for collecting fluid, according to an embodiment. The system 500 includes a fluid collection device 501 operably coupled to one or more of a fluid storage container 519 or a vacuum source 529, such as via the conduit(s) 108. The conduit 108 may fluidly connect an interior chamber of the fluid collection device 501 with the fluid storage container 519 or the vacuum source 529 (via the fluid storage container 519). Accordingly, fluids may be removed from the interior chamber via the conduit 108 via one or more of gravity feed or vacuum suction.

The fluid collection device 501 may be similar or identical to any of the fluid collection devices disclosed herein. For example, the fluid collection device 501 may include a fluid impermeable barrier, a fluid permeable body, and any of the retention means disclosed herein. The fluid collection device 501 may include the conduit 108 including an inlet and an outlet as disclosed herein. The outlet may be fluidly coupled to the fluid storage container 519 and the inlet may be positioned in the fluid collection device 501.

The conduit 108 is coupled to and at least partially extends between one or more of the fluid storage container 519 and the vacuum source 529. Accordingly, the vacuum source 529 may be fluidly connected to the fluid storage container 519 via the conduit 108. In an example, the conduit 108 is directly connected to the vacuum source 529. In some examples, the conduit 108 may be indirectly connected to at least one of the fluid storage container 519 and the vacuum source 529. In some examples, the conduit 108 may be secured to a wearer's skin with a catheter securement device, such as a STATLOCK^{®} catheter securement device available from C. R. Bard, Inc., including but not limited to those disclosed in U.S. Patent Nos. 6,117,163; 6,123,398; and 8,211,063.

Fluid (*e.g*., urine or other bodily fluids) collected in the fluid collection device 501 may be removed from the fluid collection device 501 via the conduit(s) 108. Vacuum or suction force may be applied to remove fluid from the fluid collection device via the conduit either directly or indirectly. The vacuum force may be applied indirectly via the fluid storage container 519. For example, the second open end of the conduit 108 may be disposed within the fluid storage container 519 and an additional conduit 108 may extend from the fluid storage container 519 to the vacuum source 529. Accordingly, the vacuum source 529 may indirectly apply vacuum or suction force into the fluid collection device 501 (*e.g.,* chamber therein) via the fluid storage container 519 and conduit(s) 108. As the fluid is drained from the interior chamber of the fluid collection device 501, the fluid may travel through the first section of conduit 108 to the fluid storage container 519 where it may be retained.

The fluid storage container 519 is constructed to store fluids therein. The fluid storage container may include a bag (*e.g.,* drainage bag), a rigid bottle or cup (*e.g.,* collection jar), or any other enclosed container for storing bodily fluids. The fluid storage container(s) may be fluidly connected to the vacuum source(s) 529, such as via a portion of conduit 108. The vacuum source(s) 529 provides a vacuum for pulling fluids from one or more of the fluid collection device 501 into the fluid storage container 519 via the conduit 108. The fluid collected in the fluid collection device 501 is moved through the conduit into the fluid storage container. By having a separate connection to the vacuum source 529 on the fluid storage container 519, the fluids removed from the fluid collection device 501 may be prevented from entering the vacuum source 529.

The vacuum source may include one or more of a manual vacuum pump, an electric vacuum pump, a diaphragm pump, a centrifugal pump, a displacement pump, a magnetically driven pump, a peristaltic pump, or any pump configured to produce a vacuum. The vacuum source may include a wall mounted suction line, such as found in a hospital room. In examples, the vacuum source may be powered by one or more of a power cord (*e.g.,* connected to a power socket), one or more batteries, or even manual power (*e.g.,* a hand operated vacuum pump). The vacuum source may include one or more of a switch, a button, a plug, a remote, or any other actuator suitable to activate the vacuum source. The vacuum source may be selectively operated by a user (*e.g*., medical personnel, the wearer, or a caretaker).

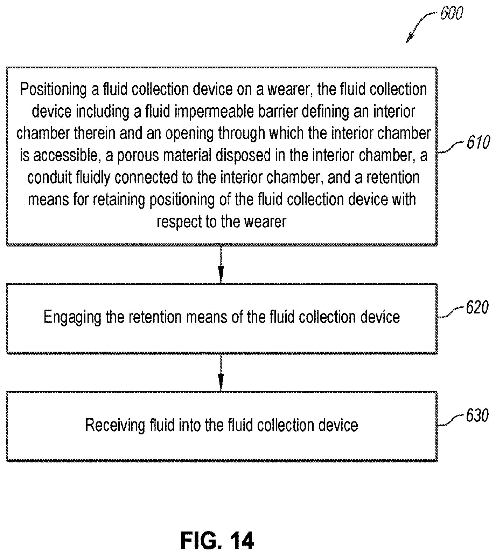

Any of the fluid collection devices and systems disclosed herein may be utilized to collect fluid, such as urine, from a wearer of the fluid collection device. **FIG. 14** **is** a flow diagram of a method 600 for collecting fluid, according to an embodiment. The method 600 includes the act 610 of positioning a fluid collection devices on a wearer, the fluid collection device including a fluid impermeable barrier defining an interior chamber therein and an opening through which the interior chamber is accessible; a porous material disposed in the interior chamber; a conduit fluidly connected to the interior chamber; and a retention means for retaining positioning of the fluid collection device with respect to the wearer. The method 600 includes the act 620 of engaging the retention means. The method 600 includes the act 630 of receiving fluid in the fluid collection device. Any the acts 610, 620, or 630 of the method 600 may be performed in different orders, split into multiple acts, modified, supplemented, or combined. For example, the acts 610 and 620 may be combined into a single act. In some examples, one or more of the blocks 610-630 of the method 600 may be omitted or additional acts may be added.

Any of the fluid collection devices disclosed herein may be used in the method 600. For example, the act 610 may include positioning any of the fluid collection devices disclosed herein on a wearer in a position to collect urine therefrom. The fluid collection device includes a fluid impermeable barrier defining an interior chamber therein and an opening through which the interior chamber is accessible, a porous material disposed in the interior chamber, a conduit fluidly connected to the interior chamber, and a retention means for retaining positioning of the fluid collection device with respect to the wearer. The retention means of the fluid collection device may include one or more of any of the retention means disclosed herein.

The act 610 of positioning a fluid collection devices on a wearer may include positioning the opening of the fluid collection device over, near, or on the urethra of the wearer. Positioning the fluid collection device on a wearer may include positioning the opening on, around, or over the labia or vulva of the wearer. Positioning the fluid collection device on a wearer (e.g., positioning the opening adjacent to a urethra of a wearer) may include positioning the second end region of the fluid collection device against or near the perineal region of the wearer with the first end region against or near the pubic region of the wearer.

The act 620 of engaging the retention means may include engaging any of the retention means disclosed herein. The act 620 of engaging the retention means may include manipulating the retention means to engage with anatomical features of the wearer to retain the fluid collection device on the wearer. For example, engaging the retention means may include manipulating the retention means to cause at least a portion of the fluid collection device to complement the shape of the anatomy of the wearer, such as the shape in the sagittal plane from the perineal region to the pubic region of the wearer, the shape of the labia of the wearer, the space between the legs of the wearer, or the like.

In examples where the retention means includes a deformable conduit (FIGS. 2-6), engaging the retention means may include bending or shaping the deformable conduit to complement the shape of the anatomy of the wearer (such as the shape in the sagittal plane from the perineal region to the pubic region of the wearer). For example, where the deformable conduit includes gooseneck tubing having tubing disposed thereover, engaging the retention means may include manually manipulating the gooseneck tubing into a selected shape such as an arcuate shape to conform to the anatomy of the wearer along the sagittal plane from the pubic region to the perineal region of the wearer. In such examples, the opening of the fluid collection device is located over the urethra of the wearer and maintained there at least in part due to the shape of the fluid collection device that is maintained by the gooseneck tubing (which includes a fit and dimension between sections of the gooseneck tubing that allows the gooseneck tubing to maintain a shape unless a force of a selected magnitude is applied thereto). The tubing disposed over the gooseneck tubing also prevents a wearer from feeling the sections of gooseneck tubing, while also allowing urine or other fluids to be removed from the tubing, such as via vacuum.

Where the deformable conduit includes gooseneck tubing having tubing disposed thereover, engaging the retention means may include manually manipulating the gooseneck tubing into a selected shape such as an arcuate shape to conform to the shape of the wearer along the sagittal plane from the pubic region to the perineal region of the wearer. In such examples, the opening of the fluid collection device is located over the urethra of the wearer and maintained there at least in part due to the shape of the fluid collection device that is maintained by the gooseneck tubing (which includes a fit and dimension between sections of the gooseneck tubing that allows the gooseneck tubing to maintain a shape unless a force of a selected magnitude is applied thereto).

In examples, where the deformable conduit includes a plurality of fibers disposed between an inner tubing and an outer tube, engaging the retention means may include bending the deformable conduit to a selected shape. The deformable conduit with a plurality of fibers therein may be bent into a selected shape such as an arcuate shape to conform to the shape of the wearer along the sagittal plane from the pubic region to the perineal region of the wearer. In such examples, the opening of the fluid collection device is located over the urethra of the wearer and maintained there at least in part due to the shape of the fluid collection device that is maintained by the plurality of fibers. For example, as the deformable conduit is bent, the plurality of fibers may move past each other and the friction therebetween may hold them in the new position to prevent spring back.

In some examples, the retention means is at least partially defined by the porous material and the porous material includes a resilient foam that is compressible (FIG. 7). For example, the resilient foam may include a memory foam. In examples with resilient foam, engaging the retention means of the fluid collection device may include one or more of compressing, shaping, or molding the fluid collection device to a compressed state and allowing the fluid collection device to at least partially expand against the anatomy of the wearer. The fluid collection device may be biased into the anatomy of the wearer, such as with the opening over the urethra of the wearer and one or more portion of the fluid collection device may be allowed to expand to conform to the anatomy of the wearer. Accordingly, the fluid collection device may be held in place by conforming to the anatomy of the wearer, such as the labia, inner thighs, or other portions of the wearer. Such resilient foams are both porous to allow fluids to move therethrough and provide a comfortable fit between wearer and the device that does not cause sores. Engaging the retention means may include compressing the fluid collection device, disposing it in an undergarment of the wearer with the opening over the urethra, and allowing, the device to at least partially expand to the anatomy of the wearer.

In some examples, engaging the retention means includes manipulating the fluid collection device to pinch or bias against a portion of the anatomy of the wearer. In such examples, the retention means may be at least partially defined by the fluid impermeable barrier. For example, the fluid impermeable barrier defines labial flanges and lobes extending therefrom (FIGS. 9-10). In such examples, the fluid impermeable barrier converges to an apex along a longitudinal axis of a wearer facing side thereof. The labial flanges include at least semi-rigid longitudinally extending flanges having valleys sized and shaped to at least partially accommodate labia therein. The lobes extend longitudinally along at least a portion of a back side of the fluid impermeable barrier and include a valley therebetween. The lateral ends of the labial flanges rotate away from the apex when the lobes are pinched due to tension applied across the fluid impermeable barrier. Engaging the retention means of the fluid collection device may include bending or pinching the lobes of the fluid collection device and releasing the lobes when the labial flanges thereof are positioned on the labia of the wearer. As the lobes are released, the labial flanges may bias inward against the labia to compress against (e.g., pinch) the labia. Accordingly, the fluid collection device may be retained in place on the wearer by the labial flanges and apex. By locating the opening on the apex, the opening may be maintained in position over the urethra of the wearer.

In some examples, engaging the retention means may include adhering a portion of the fluid collection device to the wearer. For example, the retention means may include a mucoadhesive disposed on an outer surface of the fluid impermeable barrier in one or more regions thereon (FIGS. 11 and 12). In some examples, the one or more regions include a region bordering the opening , such as around one or more portions of the opening. Engaging the retention means includes placing the mucoadhesive of the fluid collection device in contact with the wearer, such as in contact with the inner thigh, labia, pubic region, perineal region, or the like.

In some examples, a combination of any of the retention means disclosed herein may be used together. In such examples, the engaging the retention means may include engaging multiple retention means as disclosed herein.

Receiving fluid in the fluid collection device may include receiving urine or any other fluid discharged from the wearer into the fluid collection device. The urine may be received by the porous material (such as passing from the fluid permeable membrane to the fluid permeable support). For example, receiving fluid into the fluid collection device includes receiving urine from a urethra of the wearer into the interior chamber, such as into the porous material through the opening. Receiving the fluid may include receiving the fluid into a reservoir of the fluid collection device. The fluid may be removed from the interior chamber, such as from the reservoir via the conduit therein.

The devices, systems, and methods disclosed herein provide for retention of the position of fluid collection devices with respect to the anatomy (e.g., urethra) of the wearer, thereby preventing spills of bodily fluids and soiling of beds and clothing. Such improved retention of position limits the amount of monitoring of immobile or bedbound patients to ensure the fluid collection device is in position. Such improvements limit the time that care or medical professionals need to devote for ensuring urine is not contaminating the patient, undergarments, clothing, bedding, or the like.

The method 600 may include removing the fluid from the fluid collection device via the conduit. Removing the fluid from the fluid collection device via the conduit may include applying a vacuum in the interior chamber, such as into the reservoir via the conduit. Removing the fluid from the fluid collection device via the conduit may include allowing fluid to be removed from interior chamber via gravity. Removing the fluid from the fluid collection device via the conduit may include removing the fluid into a fluid storage container, such as via vacuum or gravity feed.

While various aspects and embodiments have been disclosed herein, other aspects and embodiments are contemplated. The various aspects and embodiment disclosed herein are for purposes of illustration and are not intended to be limiting. Features from any of the disclosed embodiments may be used in combination with one another, without limitation.

## Claims

1. A fluid collection device (100), the fluid collection device comprising:
a fluid impermeable barrier (102) defining an interior chamber therein, an aperture, and an opening (106) through which the interior chamber is accessible;
a porous material (115) disposed in the interior chamber; and
a deformable conduit (108a) comprising:
first tubing (109) fluidly connected to the interior chamber and extending through the aperture; and
second tubing (110) disposed in or on the first tubing, the second tubing configured to retain a position of the fluid collection device with respect to the wearer, wherein the second tubing includes gooseneck tubing,
**characterised in that** the second tubing is not fluid tight, and **in that** the gooseneck tubing extends only along a portion of the first tubing that is inside the interior chamber.

2. The fluid collection device of claim 1, wherein the first tubing includes flexible tubing.

3. The fluid collection device of claim 2, wherein the flexible tubing includes an inlet disposed in the second end region and an outlet spaced from the interior chamber.

4. The fluid collection device of claim 1, wherein the fluid impermeable barrier including a first end region (125) and a second end region (127) opposite the first end region, the aperture at the first end region and a reservoir (122) at the second end region, the first tubing extending through the aperture at the first end region.

5. The fluid collection device of claim 1, wherein the gooseneck tubing defines an interior passageway configured to allow fluid to flow therethrough.

6. The fluid collection device of claim 1, wherein the gooseneck tubing is disposed in the first tubing.

7. The fluid collection device of claim 1, wherein the porous material defines a space that accommodates at least the first tubing and the second tubing, the second tubing disposed only in the space defined by the porous material.

8. The fluid collection device of claim 1, wherein the fluid impermeable barrier defines a reservoir (122) and the gooseneck tubing is spaced from the reservoir.

9. The fluid collection device of claim 1, wherein the gooseneck tubing is positioned concentrically relative to the flexible tubing.

10. The fluid collection device of claim 9, wherein the first tubing includes flexible tubing.

11. The fluid collection device of claim 1, wherein the gooseneck tubing exhibits a diameter of about 5 mm to about 13 mm and a diameter of a deformable conduit comprising the first tubing and the second tubing is about 5 mm to about 13 mm.

12. The fluid collection device of claim 1, wherein the second tubing includes a plurality of fibers (111) and an outer tube, the plurality of fibers positioned between the first tubing and the outer tube.

## Patentansprüche

1. Flüssigkeitssammelvorrichtung (100), wobei die Flüssigkeitssammelvorrichtung umfasst:
eine flüssigkeitsundurchlässige Barriere (102), die darin eine Innenkammer, eine Aussparung und eine Öffnung (106) definiert, durch die die Innenkammer zugänglich ist;
ein poröses Material (115), das in der Innenkammer angeordnet ist; und
eine verformbare Leitung (108a), umfassend:
einen ersten Schlauch (109), der mit der Innenkammer in Flüssigkeitsverbindung steht und sich durch die Aussparung erstreckt; und
einen zweiten Schlauch (110), der in oder an dem ersten Schlauch angeordnet ist, wobei der zweite Schlauch ausgebildet ist, um eine Position der Flüssigkeitssammelvorrichtung in Bezug auf den Träger beizubehalten, wobei der zweite Schlauch einen Schwanenhalsschlauch einschließt, **dadurch gekennzeichnet, dass** der zweite Schlauch nicht flüssigkeitsdicht ist und dass sich der Schwanenhalsschlauch nur entlang eines Abschnitts des ersten Schlauchs erstreckt, der innerhalb der Innenkammer ist.

2. Flüssigkeitssammelvorrichtung nach Anspruch 1, wobei der erste Schlauch einen flexiblen Schlauch einschließt.

3. Flüssigkeitssammelvorrichtung nach Anspruch 2, wobei der flexible Schlauch einen Einlass einschließt, der in dem zweiten Endbereich angeordnet ist, und einen Auslass, der von der Innenkammer beabstandet ist.

4. Flüssigkeitssammelvorrichtung nach Anspruch 1, wobei die flüssigkeitsundurchlässige Barriere einen ersten Endbereich (125) und einen dem ersten Endbereich gegenüberliegenden zweiten Endbereich (127) mit der Aussparung an dem ersten Endbereich und einem Reservoir (122) an dem zweiten Endbereich einschließt, wobei sich der erste Schlauch durch die Aussparung an dem ersten Endbereich erstreckt.

5. Flüssigkeitssammelvorrichtung nach Anspruch 1, wobei der Schwanenhalsschlauch einen Innenkanal definiert, der ausgebildet ist, um zu ermöglichen, dass Flüssigkeit hindurchströmt.

6. Flüssigkeitssammelvorrichtung nach Anspruch 1, wobei der Schwanenhalsschlauch in dem ersten Schlauch angeordnet ist.

7. Flüssigkeitssammelvorrichtung nach Anspruch 1, wobei das poröse Material einen Raum definiert, der zumindest den ersten Schlauch und den zweiten Schlauch aufnimmt, wobei der zweite Schlauch nur in dem durch das poröse Material definierten Raum angeordnet ist.

8. Flüssigkeitssammelvorrichtung nach Anspruch 1, wobei die flüssigkeitsundurchlässige Barriere ein Reservoir (122) definiert und der Schwanenhalsschlauch von dem Reservoir beabstandet ist.

9. Flüssigkeitssammelvorrichtung nach Anspruch 1, wobei der Schwanenhalsschlauch relativ zu dem flexiblen Schlauch konzentrisch positioniert ist.

10. Flüssigkeitssammelvorrichtung nach Anspruch 9, wobei der erste Schlauch einen flexiblen Schlauch einschließt.

11. Flüssigkeitssammelvorrichtung nach Anspruch 1, wobei der Schwanenhalsschlauch einen Durchmesser von etwa 5 mm bis etwa 13 mm aufweist und ein Durchmesser einer verformbaren Leitung, die den ersten Schlauch und den zweiten Schlauch umfasst, etwa 5 mm bis etwa 13 mm beträgt.

12. Flüssigkeitssammelvorrichtung nach Anspruch 1, wobei der zweite Schlauch eine Vielzahl von Fasern (111) und einen Außenschlauch einschließt, wobei die Vielzahl von Fasern zwischen dem ersten Schlauch und dem Außenschlauch positioniert ist.

## Revendications

1. Dispositif (100) de collecte de fluide, le dispositif de collecte de fluide comprenant :
une barrière (102) imperméable aux fluides définissant en son sein une chambre intérieure, un orifice et une ouverture (106) à travers laquelle la chambre intérieure est accessible ;
un matériau poreux (115) disposé dans la chambre intérieure ; et
un conduit déformable (108a) comprenant :
une première tubulure (109) en communication fluidique avec la chambre intérieure et s'étendant à travers l'orifice ; et
une seconde tubulure (110) disposée dans ou sur la première tubulure, la seconde tubulure étant configurée pour maintenir la position du dispositif de collecte de fluide par rapport au porteur, la seconde tubulure incluant une tubulure en col de cygne, **caractérisé en ce que** la seconde tubulure n'est pas étanche aux fluides et **en ce que** la tubulure en col de cygne s'étend uniquement le long d'une portion de la première tubulure qui est à l'intérieur de la chambre intérieure.

2. Dispositif de collecte de fluide selon la revendication 1, dans lequel la première tubulure inclut une tubulure flexible.

3. Dispositif de collecte de fluide selon la revendication 2, dans lequel la tubulure flexible inclut une entrée disposée dans la seconde région d'extrémité et une sortie espacée de la chambre intérieure.

4. Dispositif de collecte de fluide selon la revendication 1, dans lequel la barrière imperméable aux fluides inclut une première région d'extrémité (125) et une seconde région d'extrémité (127) opposée à la première région d'extrémité, l'orifice au niveau de la première région d'extrémité et un réservoir (122) au niveau de la seconde région d'extrémité, la première tubulure s'étendant à travers l'orifice au niveau de la première région d'extrémité.

5. Dispositif de collecte de fluide selon la revendication 1, dans lequel la tubulure en col de cygne définit un passage intérieur configuré pour permettre au fluide de s'écouler à travers celui-ci.

6. Dispositif de collecte de fluide selon la revendication 1, dans lequel la tubulure en col de cygne est disposée dans la première tubulure.

7. Dispositif de collecte de fluide selon la revendication 1, dans lequel le matériau poreux définit un espace qui reçoit au moins la première tubulure et la seconde tubulure, la seconde tubulure étant disposée seulement dans l'espace défini par le matériau poreux.

8. Dispositif de collecte de fluide selon la revendication 1, dans lequel la barrière imperméable aux fluides définit un réservoir (122) et la tubulure en col de cygne est espacée du réservoir.

9. Dispositif de collecte de fluide selon la revendication 1, dans lequel la tubulure en col de cygne est positionnée de manière concentrique par rapport à la tubulure flexible.

10. Dispositif de collecte de fluide selon la revendication 9, dans lequel la première tubulure comprend une tubulure flexible.

11. Dispositif de collecte de fluide selon la revendication 1, dans lequel la tubulure en col de cygne présente un diamètre d'environ 5 mm à environ 13 mm et un diamètre d'un conduit déformable comprenant la première tubulure et la seconde tubulure est d'environ 5 mm à environ 13 mm.

12. Dispositif de collecte de fluide selon la revendication 1, dans lequel la seconde tubulure inclut une pluralité de fibres (111) et un tube externe, la pluralité de fibres étant positionnée entre la première tubulure et le tube externe.
